# EUROPEAN PATENT APPLICATION

(11) **EP 1 797 872 A1**
(43) Date of publication of application: **20.06.2007**
(21) Application number: 06250761.1
(22) Date of filing: 13.02.2006
(51) Int. Cl.: A61K 9/20, A61K 31/4184

(54) **Telmisartan-containing pharmaceutical compositions for oral intake**

(30) Priority: 22.11.2005 US 286017
(71) Applicant: Teva Pharmaceutical Industries Ltd, 49131 Petah Tiqva (IL)
(72) Inventor: Kolatkar, Gershon, Petah Tiqva 49275 (IL); Zisman, Erela, Rishon le Zion 75357 (IL)
(74) Representative: Nachshen, Neil Jacob

(57) **Abstract**

The present invention provides a pharmaceutical composition of telmisartan comprising a) a telmisartan compound, b) a surfactant, c) a basic agent, and d) at least one diluent wherein the composition comprises less than 25% of water soluble diluents. The telmisartan compound is preferably present from about 12.5% to about 15.5%. At least one water insoluble diluent is preferred for use in the pharmaceutical composition in an amount from about 40% to about 70% of the total weight of the pharmaceutical composition. A preferred water insoluble diluent is microcrystalline cellulose.

## Description

### FIELD OF THE INVENTION

The present invention is directed to pharmaceutical compositions of telmisartan having less than 25% of water soluble diluents.

### BACKGROUND OF THE INVENTION

Telmisartan is the common chemical name for the compound 4'-[2-n-propyl-4-methyl-6-(1-methylbenzimidazol-2-yl)benzimidazol-1-ylmethyl]biphenyl-2-carboxylic acid. (CAS Registry No.144701-48-4.) The empirical formula of telmisartan is C₃₃H₃₀N₄O₂ and its molecular weight is 514.63. The molecular structure of telmisartan is represented by Formula I.

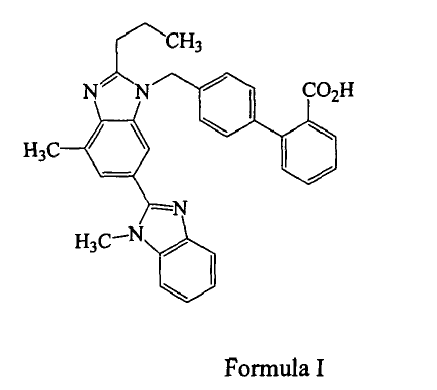

Telmisartan is a non-peptide angiotensin II receptor (type AT₁) antagonist. The United States Food and Drug Administration (FDA) approved it for the treatment of hypertension. It may be used alone or in combination with other hypertensive agents, such as hydrochlorothiazide. Boehringer Ingelheim markets telmisartan under the trade name Micardis^{®} (telmisartan), available as 20, 40 and 80 mg tablets for oral administration¹. Two patents are listed in the FDA's electronic Orange Book for telmisartan, U.S. Patent No. 6,358,986 ("the '986 patent") and U.S. Patent No. 5,591,762 ("the '762 patent").
¹ The Prescribing Information available at http://us.micardis.com/bipi/InformationRequest?page=prescribing&product=micardis describes the 20mg, 40mg and 80mg tablets as contain the following inactive ingredients: sodium hydroxide, meglumine, povidone, sorbitol and magnesium stearate.

The '986 patent apparently describes that telmisartan and the physiologically acceptable salts thereof can also be used to treat cardiac insufficiency, ischaemic peripheral circulatory disorders, myocardial ischaemia (angina), diabetic neuropathy, glaucoma, gastrointestinal diseases, bladder diseases, and to prevent progression of cardiac insufficiency after myocardial infarct.

In addition to the above therapeutic applications of telmisartan, the '762 patent discloses other therapeutic applications, including treating diabetic nephropathy, pulmonary diseases, *e.g.*, lung oedema and chronic bronchitis. It also discloses using telmisartan to prevent arterial restenosis after angioplasty, thickening of blood vessel walls after vascular operations, and diabetic angiopathy. The '762 patent further discloses using telmisartan to alleviate central nervous system disorders, such as depression, Alzheimer's disease, Parkinson Syndrome, bulimia, and disorders of cognitive function in view of the effects of angiotensin on the release of acetylchloline and dopamine in the brain.

Telmisartan is a white to off-white, odorless crystalline powder. It is practically insoluble in water or an aqueous solution in the pH range of 3 to 9, and sparingly soluble in a strong acid, with the exception of hydrochloric acid in which it is insoluble. Telmisartan is soluble in a strong base.

In general, Telmisartan is manufactured and supplied in its free acid form. However, as described in WO 00/43370, crystalline Telmisartan apparently exists in two polymorphic forms which have different melting points. Under the influence of heat and humidity polymorph B of Telmisartan, having the lower melting point, reportedly irreversibly transforms into polymorph A, which has a higher melting point. Both of these forms are apparently very poorly soluble in aqueous systems at the physiological pH range in the gastro-intestinal tract of between pH 1 to 7.

The published U.S. patent application 2004/0110813 A1, describes that the solubility of Telmisartan can be increased several hundred fold in a pharmaceutical composition comprising 3 to 50% of Telmisartan dispersed in a dissolving matrix comprising a) a basic agent wherein the molar ratio of basic agent: Telmisartan equals 1:1 to 10:1, b) a surfactant or emulsifier in an amount of about 2 to 3% of the final composition, c) 25 to 70% of a water soluble diluent, and optionally 0 to 20% of further excipients and/or adjuvants.

The present invention is directed to pharmaceutical compositions comprising telmisartan in which the solubility of telmisartan is improved, and wherein the composition comprises less than 25% of water soluble diluents.

### SUMMARY OF THE INVENTION

The present invention provides a pharmaceutical composition comprising a) a telmisartan compound, b) a surfactant, c) a basic agent, and d) at least one diluent selected from water soluble and water insoluble diluents, wherein the pharmaceutical composition comprises less than 25% by total weight of the composition of water soluble diluents. Preferably the pharmaceutical composition comprises at least one diluent which is water insoluble, preferably microcrystalline cellulose.

Unexpectedly, it was determined that telmisartan containing pharmaceutical compositions of the present invention provide sufficient solubility of telmisartan for use in a physiological environment while the amount of water soluble diluents is less than 25% of the composition by weight. Telmisartan is released from said pharmaceutical compositions with sufficient solubility for gastro-intestinal absorption in the slightly acidic and neutral pH region. In one embodiment of the invention dissolution of the pharmaceutical composition in an aqueous solution of neutral pH dissolves at least 80% of the Telmisartan contained therein within 45 minutes. Preferably, at least 80% of the Telmisartan is dissolved from the pharmaceutical composition in such aqueous solution within 30 minutes, and most preferably at least 80% of the Telmisartan is dissolved from the pharmaceutical composition in such aqueous solution within 20 minutes.

In another embodiment of the present invention there is provided a pharmaceutical composition comprising about 12.5% to about 15.5% weight percent of telmisartan; about 40% to about 70% weight percent microcrystalline cellulose; about 2.0% to about 3.5% weight percent of a surfactant, preferably Poloxamer 188; about 9% to about 12% weight percent of a basic agent, preferably Meglumine; about 1.0% to about 1.5% weight percent of a binder; about 7.5% to about 10% weight of a disintegrant; about 0% to 17% weight percent of a water soluble filler and about 0.5% to about 1% weight of a lubricant, all weight percentages are based upon the total weight of the pharmaceutical composition.

Further there is provided a method of preparing a pharmaceutical composition comprising the following steps of 1) mixing a disintegrant, preferably sodium starch glycolate, and one or more diluents, preferably at least one water insoluble diluent, more preferably wherein at least one diluent is microcrystalline cellulose, in a high shear mixer to form a homogeneous mixture; 2) preparing a granulation suspension of purified water, alcohol, a basic agent, a surfactant, and telmisartan; 3) combining the homogeneous mixture and the granulation suspension to form a combined mixture; 4) preparing a granulation solution of water and a binder, preferably Povidone (preferably PVP K-30); 5) adding the granulation solution to the combined mixture to form a granulate; 6) drying the formed granules; 7) sizing the dried granules; 8) mixing the dried granulate with a disintegrant, preferably sodium starch glycolate, and optionally a filler, preferably sorbitol, and; 9) adding a lubricant, preferably magnesium stearate; and 10) compressing the granules into tablets, wherein the prepared pharmaceutical composition comprises less than 25% by weight of water soluble diluents. Further, when the pharmaceutical composition to be prepared is a capsule as opposed to tablets, step 10 of the method is replaced by a step of filling capsules.

In another embodiment of the present invention there is provided a method of treating a patient suffering from hypertension comprising administering an effective amount of a telmisartan compound in a pharmaceutical composition comprising about 12.5% to about 15.5% of a telmisartan compound; about 2.0 to about 3.5% of a surfactant; about 9% to about 12% of a basic agent; and at least one diluent, wherein the pharmaceutical composition comprises less than 25% of water soluble diluents. Preferably, the pharmaceutical composition comprises about 40% to about 70% of a water insoluble diluent.

### DETAILED DESCRIPTION OF THE INVENTION

As used throughout this specification, the term water soluble diluent refers to a compound, used in the art as a diluent, for use in a pharmaceutical composition which is soluble in an aqueous environment. In contrast, the term water insoluble diluent refers to a compound, used in the art as a diluent, for use in a pharmaceutical composition which is insoluble or very poorly soluble in an aqueous environment.

The pharmaceutical composition according to the invention comprises:
a) a telmisartan compound, in admixture with
b) a surfactant,
c) a basic agent, and
d) at least one diluent selected from water soluble and water insoluble diluents, wherein the amount of water soluble diluents in the pharmaceutical composition is less than 25% by weight of the pharmaceutical composition. Preferably, the amount of water soluble diluents in the pharmaceutical composition is less than 20%, more preferably less than 17.5%, by weight of the pharmaceutical composition. Said pharmaceutical composition preferably comprises at least one water insoluble diluent.

Preferably, a pharmaceutical composition of the invention is in tablet dosage form containing telmisartan as the active ingredient in an amount of 20, 40 or 80 mg in each tablet, and wherein the total weight of the pharmaceutical composition is from about 130mg to about 160mg. More preferably, such tablets contain 20 mg of telmisartan. The relative amount of the telmisartan compound typically varies from about 12.5% to about 15.5% by weight of the pharmaceutical composition. Further, telmisartan as used in the pharmaceutical composition of the present invention may be in any suitable form such as for example in the form of polymorph A or B.

Suitable diluents for use in the pharmaceutical composition of the invention include microcrystalline cellulose (e.g. Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, potassium chloride, powdered cellulose, sodium chloride, sorbitol and talc. However, the total amount of water soluble diluents in the pharmaceutical composition is less than 25% by weight of the pharmaceutical composition. In a preferred embodiment of the invention at least one diluent for use in the pharmaceutical composition is a water insoluble diluent such as microcrystalline cellulose. Preferably, the insoluble diluent for use in the pharmaceutical composition of the invention is microcrystalline cellulose (e.g. Avicel^{®}PH 102). In such prefered embodiment of the invention, the relative amount of the water insoluble diluent is preferably from about 40% to about 70% by weight of the pharmaceutical composition.

Suitable surfactants for use in the pharmaceutical composition of the invention include poloxamers, polyethylene glycols, polysorbates, sodium lauryl sulfate, polyethoxylated castor oil, and hydroxylated castor oil. A preferred surfactant for use in the pharmaceutical composition of the invention is Poloxamer 188. Preferably, the pharmaceutical composition comprises an amount of about 2.0% to about 3.5% by total weight of the composition of the surfactant.

Suitable basic agents for use in the pharmaceutical composition of the invention include alkaline hydroxides, alkaline carbonates, alkaline phosphates, basic amino acids, and Meglumine. A preferred basic agent for use in the pharmaceutical composition of the invention is Meglumine. Preferably, the pharmaceutical composition comprises an amount of about 9% to about 12% by total weight of the composition of the basic agent.

The telmisartan pharmaceutical composition of the invention may also include pharmaceutically acceptable excipients. As is well known to those skilled in the art, pharmaceutical excipients are routinely incorporated into solid dosage forms. This is done to ease the manufacturing process as well as to improve the performance of the dosage form. Common excipients include fillers, binders, lubricants, etc. Such excipients could be used in the dosage forms of this invention.

Fillers, are added in order to increase the mass of an individual dose to a size suitable for tablet compression. Suitable fillers include for example powdered sugar, calcium phosphate, calcium sulfate, microcrystalline cellulose, lactose, mannitol, kaolin, sodium chloride, dry starch, sorbitol, and talc. A water soluble filler may optionally be used. A preferred filler of this type for use in the pharmaceutical composition of the invention is sorbitol in an amount not exceeding 25% by total weight of the composition. Preferably, the pharmaceutical composition comprises an amount of about 0% to about 17% by total weight of the composition of the optional filler.

Solid pharmaceutical compositions that are compacted into a dosage form, such as a tablet, may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydroxyethyl cellulose, hydroxypropyl cellulose (e.g. Klucel^{®}), hydroxypropyl methyl cellulose (e.g. Methocel^{®}), liquid glucose, maltodextrin, methylcellulose, polymethacrylates, povidone (e.g. Povidone PVP K-30, Kollidon^{®}, Plasdone^{®}), pregelatinized starch, sodium alginate and starch. A preferred binder for use in the pharmaceutical composition of the present invention is Povidone (e.g. PVP K-30). Preferably, the pharmaceutical composition comprises an amount of about 1.0% to about 1.5% by total weight of the composition of the binder.

A compacted solid pharmaceutical composition may also include the addition of a disintegrant to the composition. Disintegrants include croscarmellose sodium (e.g. Ac Di Sol^{®}, Primellose^{®}), crospovidone (e.g. Kollidon^{®}, Polyplasdone^{®}), microcrystalline cellulose, polacrilin potassium, powdered cellulose, pregelatinized starch, sodium starch glycolate (e.g. Explotab^{®}, Primoljel^{®}) and starch. A preferred disintegrant for use in the pharmaceutical composition of the invention is sodium starch glycolate. Preferably, the pharmaceutical composition comprises an amount of about 7.5% to about 10% by total weight of the composition of the disintegrant.

Glidants can be added to improve the flowability of a non compacted solid composition and to improve the accuracy of dosing. Excipients that may function as glidants include colloidal silicon dioxide, magnesium trisilicate, powdered cellulose, and talc.

A lubricant can be added to the composition to reduce adhesion and/or ease the release of the product from e.g. the dye. Lubricants include magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc and zinc stearate. A preferred lubricant for use in the pharmaceutical composition of the invention is magnesium stearate. Preferably, the pharmaceutical composition comprises an amount of about 0.5% to about 1.0% by total weight of the composition of the lubricant.

Other excipients that may be incorporated into the formulation include preservatives, antioxidants, or any other excipient commonly used in the pharmaceutical industry.

The solid compositions of the present invention include powders, granulates, aggregates and compacted compositions. The dosages include dosages suitable for oral, buccal, and rectal administration. Although the most suitable administration in any given case will depend on the nature and severity of the condition being treated, the most preferred route of the present invention is oral. The dosages may be conveniently presented in unit dosage form and prepared by any of the methods well known in the pharmaceutical arts.

Dosage forms include solid dosage forms like tablets, powders, capsules, sachets, troches and losenges.

The dosage form of the present invention may be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

Particularly preferred pharmaceutical compositions of the invention are described in Table 1 infra, wherein all weight percentages are based upon the total weight of the composition.

**Table 1: Preferred Pharmaceutical Compositions of the Invention**

| Ingredient | Component | (%) of composition |
|---|---|---|
| Telmisartan | Active Drug | 12.5-15.5 |
| Microcrystalline Cellulose (Avicel PH 102) | Water Insoluble Diluent | 40-70 |
| Poloxamer 188 | Surfactant | 2.0-3.5 |
| Meglumine | Basic Agent | 9-12 |
| Povidone (PVP K-30) | Binder | 1.0-1.5 |
| Sodium starch glycolate | Disintegrant | 7.5-10 |
| Sorbitol | Filler | 0-17 |
| Magnesium Stearate | Lubricant | 0.5-1.0 |

The solubility of telmisartan is sufficient for gastro-intestinal absorption from such pharmaceutical compositions of telmisartan in the slightly acidic and neutral pH region. Telmisartan dissolves from the pharmaceutical composition of the present invention at a suitable rate. Preferably, at least 80% of the telmisartan in the pharmaceutical composition dissolves in a neutral aqueous environment within 45 minutes. More preferably, at least 80% of the telmisartan in the pharmaceutical composition dissolves in a neutral aqueous environment within 30 minutes, and most preferably at least 80% of the Telmisartan is dissolved from the pharmaceutical composition in such aqueous solution within 20 minutes.

The active ingredient and excipients may be formulated into compositions and dosage forms according to methods known in the art.

A composition for tableting or capsule filling may be prepared by wet granulation. In wet granulation, some or all of the active ingredients and excipients in powder form are blended and then further mixed in the presence of a liquid, typically water, that causes the powders to clump into granules. The granulate is screened and/or milled, dried and then screened and/or milled to the desired particle size. The granulate may then be tableted, or other excipients may be added prior to tableting, such as a glidant and/or a lubricant.

A tableting composition may be prepared conventionally by dry blending. For example, the blended composition of the actives and excipients may be compacted into a slug or a sheet and then comminuted into compacted granules. The compacted granules may subsequently be compressed into a tablet. The granulates and powder blends produced by these methods can alternatively be filled into capsules or sachets for example.

As an alternative to dry granulation, a blended composition may be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate and colloidal silica. The proper use of these and other excipients in direct compression tableting is known to those in the art with experience and skill in particular formulation challenges of direct compression tableting.

A capsule filling of the present invention may comprise any of the aforementioned blends and granulates that were described with reference to tableting, however, they are not subjected to a final tableting step.

A preferred method of preparing a telmisartan pharmaceutical composition of the invention comprises the steps of 1) mixing a disintegrant, preferably sodium starch glycolate, and one or more diluents, preferably at least one water insoluble diluent, more preferably wherein at least one diluent is microcrystalline cellulose, in a high shear mixer to form a homogeneous mixture; 2) preparing a granulation suspension of purified water, alcohol, a basic agent, a surfactant, and telmisartan; 3) combining the homogeneous mixture and the granulation suspension to form a combined mixture; 4) preparing a granulation solution of water and a binder, preferably Povidone (e.g. PVP K-30); 5) adding the granulation solution to the combined mixture to form a granulate; 6) drying the formed granules; 7) sizing the dried granules; 8) mixing the dried granulate with a filler, preferably sorbitol, and a disintegrant, preferably sodium starch glycolate; 9) adding a lubricant, preferably magnesium stearate to form a final mixture; and 10) compressing the final mixture into tablets, wherein the prepared pharmaceutical composition comprises less than 25% by weight of water soluble diluents. In preparing capsules step 10 will typically be replaced by the step of filling capsule shells. The method of the present invention is however not limited to these mixing procedures or their order, and several of the steps involved can be combined into a single step.

In the above method the amount of disintegrant used preferably results in a pharmaceutical composition comprising about 7.5% to about 10% of a disintegrant. Also, the amount of a telmisartan compound used in said method preferably results in a pharmaceutical composition comprising about 12.5% to about 15.5% of a telmisartan compound. The amount of a basic agent used in said method preferably results in a pharmaceutical composition comprising about 9% to about 12% of a basic agent. The amount of a binder used in said method preferably results in a pharmaceutical composition comprising about 1.0% to about 1.5% of a binder. Further, the amount of surfactant used in said method preferably results in a pharmaceutical composition comprising about 2.0% to about 3.5% of a surfactant. The amount of an optional filler used in said method preferably results in a pharmaceutical composition comprising about 0% to about 17% of a filler. The amount of a lubricant used in said method is preferably from about 0.5% to about 1.0%.

Telmisartan is used for the treatment of hypertension in patients, either alone or in combination with other hypertensive agents. The pharmaceutical compositions of the present invention provide an effective delivery system for the administration of telmisartan to patients in need of such treatment. Treatment of hypertensive patients may comprise administering an effective amount of telmisartan in a pharmaceutical composition comprising about 12.5% to about 15% telmisartan, about 2.0 to about 3.5% of a surfactant; about 9% to about 12% of a basic agent; and at least one diluent, wherein the pharmaceutical composition comprises less than 25% of water soluble diluents. Preferably, the pharmaceutical composition comprises about 40% to about 70% of a water insoluble diluent.

The following examples are presented in order to further illustrate the invention. These examples should not be construed in any manner to limit the invention.

### EXAMPLES

### Example 1: Telmisartan formulations comprising 41 % of a water insoluble diluent.

Telmisartan tablets were prepared containing 41 % of a water insoluble diluent. Sorbitol, a filler in these formulations, may also be considered a water soluble diluent and is thus present in less than 25%, i.e. 16.7%, of the composition. The tablets were prepared in three potencies, 20, 40 and 80 mg Telmisartan tablets. The tablet compositions are presented in Table 2.

**Table 2: Telmisartan formulations comprising 41 % of a water insoluble diluent.**

| **Ingredients** | **20 mg tablet (mg/tablet)** | **40 mg tablet (mg/tablet)** | **80 mg tablet (mg/tablet)** | **Concentration (% w/w)** |
|---|---|---|---|---|
| Telmisartan | 20 | 40 | 80 | 15.4 |
| Microcrystalline Cellulose (Avicel PH 102) | 54 | 108 | 216 | 41.5 |
| Poloxamer 188 | 4 | 8 | 16 | 3.1 |
| Meglumine | 15 | 30 | 60 | 11.5 |
| Povidone (PVP K-30) | 2 | 4 | 8 | 1.5 |
| Sodium starch glycolate | 12 | 24 | 48 | 9.2 |
| Sorbitol | 21.75 | 43.5 | 87 | 16.7 |
| Magnesium Stearate | 1.25 | 2.5 | 5 | 1.0 |
| **Total** | **130.0** | **260.0** | **520.0** | **100.0** |

The tablets were prepared by mixing a homogeneous mixture of microcrystalline cellulose and sodium starch glycolate with a granulation suspension of purified water, Poloxamer 188, Meglumine, and telmisartan in a high shear mixer to form a partial granulate mixture. This partial granulate mixture was granulated with a granulation solution of alcohol and Povidone (PVP K-30). The obtained granules were dried in a Fluid Bed dryer until the observed "loss on drying" (LOD) was 2% or less. The dried granules were then milled by passing them through an oscillating granulator. Subsequently, the milled granulates were mixed with Sorbitol and sodium starch glycolate in a blender. The obtained blend was then mixed with magnesium stearate and tablets were prepared by compressing this mixture.

### Example 2: Telmisartan formulations comprising 53% of a water insoluble diluent.

Telmisartan tablets were prepared containing 53% of a water insoluble diluent. Sorbitol, a filler in these formulations, may also be considered a water soluble diluent and is thus present in an amount less than 25%, i.e. 13.4%, of the composition. The tablets were prepared in three potencies, 20, 40 and 80 mg Telmisartan tablets. The tablet compositions are presented in Table 3.

**Table 3: Telmisartan formulations comprising 53% of a water insoluble diluent.**

| **Ingredients** | **20 mg tablet (mg/tablet)** | **40 mg tablet (mg/tablet)** | **80 mg tablet (mg/tablet)** | **Concentration (% w/w)** |
|---|---|---|---|---|
| Telmisartan | 20 | 40 | 80 | 12.5 |
| Microcrystalline Cellulose (Avicel PH 102) | 84 | 168 | 336 | 52.5 |
| Poloxamer 188 | 4 | 8 | 16 | 2.5 |
| Meglumine | 15 | 30 | 60 | 9.375 |
| Povidone (PVP K-30) | 2 | 4 | 8 | 1.25 |
| Sodium starch glycolate | 12 | 24 | 48 | 7.5 |
| Sorbitol | 21.4 | 42.8 | 85.6 | 13.375 |
| Magnesium Stearate | 1.6 | 3.2 | 6.4 | 1.0 |
| **Total** | **160.0** | **320.0** | **640.0** | **100.0** |

The tablets were prepared by wet granulation by mixing a homogeneous mixture of microcrystalline cellulose and sodium starch glycolate with a granulation suspension of purified water, Poloxamer 188, Meglumine, and telmisartan in a high shear mixer to form a partial granulate mixture. This partial granulate mixture was granulated with a granulation solution of alcohol and Povidone (PVP K-30). The obtained granules were dried in a Fluid Bed dryer until the observed "loss on drying" (LOD) was 2% or less. The dried granules were then milled by passing them through an oscillating granulator. Subsequently, the milled granulates were mixed with Sorbitol and sodium starch glycolate in a blender. The obtained blend was then mixed with magnesium stearate and tablets were prepared by compressing this mixture.

### Example 3: Telmisartan formulations comprising 66% of a water insoluble diluent.

Telmisartan tablets were prepared containing 66% of a water insoluble diluent. Moreover, these formulations do not comprise water soluble diluents. The tablets were prepared in three potencies, 20, 40 and 80 mg Telmisartan tablets. The tablet compositions are presented in Table 4.

**Table 4: Telmisartan formulations comprising 66% of a water insoluble diluent.**

| **Ingredients** | **20 mg tablet (mg/tablet)** | **40 mg tablet (mg/tablet)** | **80 mg tablet (mg/tablet)** | **Concentration (% w/w)** |
|---|---|---|---|---|
| Telmisartan | 20 | 40 | 80 | 12.5 |
| Microcrystalline Cellulose (Avicel PH 102) | 105.4 | 210.8 | 421.6 | 65.875 |
| Poloxamer 188 | 4 | 8 | 16 | 2.5 |
| Meglumine | 15 | 30 | 60 | 9.375 |
| Povidone (PVP K-30) | 2 | 4 | 8 | 1.25 |
| Sodium starch glycolate | 12 | 24 | 48 | 7.5 |
| Magnesium Stearate | 1.6 | 3.2 | 6.4 | 1.0 |
| **Total** | **160.0** | **320.0** | **640.0** | **100.0** |

The tablets were prepared by wet granulation through a method analogous to the method used to prepare the tablets of example 1.

### Example 4: Dissolution tests with Telmisartan pharmaceutical tablet dosage forms.

Dissolution tests with the Telmisartan pharmaceutical tablet dosage formulations of examples 1 to 3 were performed. In these tests the dissolution of the tablets of examples 1 to 3 was compared with the dissolution of the reference tablet formulation, a Micardis^{®} tablet. These in vitro dissolution tests were conducted using an Apparatus II (Paddle Method) as described in the United State Pharmacopeia XXI/National Formulary XVI. The comparative dissolution tests were conducted under the following conditions. Micardis^{®} tablets and Telmisartan tablets of examples 1, 2, and 3 were dissolved in a USP type II apparatus at a paddle speed of 75 rpm, at a temperature of 37°C, in 900ml of a buffer at pH 7.5. The results of these dissolution tests are presented in Table 5.

**Table 5: Comparative dissolution.**

| **Time (minutes)** | **% Dissolution** | | | |
|---|---|---|---|---|
| | **Micardis®** | **Example 1** | **Example 2** | **Example 3** |
| **10** | 53 | 53 | 61 | 38 |
| **20** | 81 | 89 | 99 | 68 |
| **30** | 96 | 99 | 101 | 99 |
| **45** | 100 | 99 | 101 | 99 |
| **60** | 100 | 100 | 102 | 101 |

Based upon these dissolution studies, the results appearing in Table 5 above, it can be concluded that the pharmaceutical tablet formulations of the present invention and the reference Micardis^{®} tablet all dissolve at least 80% of the telmisartan contained therein well within the desired 45 minutes. In fact, all dissolve at least 80% of the telmisartan contained in the formulation within 30 minutes, and some formulations even achieve that at least 80% of the telmisartan from the pharmaceutical composition is dissolved in such aqueous solution within 20 minutes. The results further demonstrate that the pharmaceutical tablet formulations of the invention have a similar dissolution profile compared to the reference formulation.

## Claims

1. A pharmaceutical composition comprising:
a) a telmisartan compound, in admixture with
b) a surfactant,
c) a basic agent, and
d) at least one diluent selected from water soluble and water insoluble diluents, wherein the amount of water soluble diluents in the pharmaceutical composition is less than 25% by weight of the pharmaceutical composition.

2. The pharmaceutical composition according to claim 1, wherein the telmisaran compound comprises an amount from about 12.5% to about 15% of the total weight of the composition.

3. The pharmaceutical composition according to claim 1 or 2, wherein at least one diluent is a water insoluble diluent.

4. The pharmaceutical composition according to claim 3, wherein the water insoluble diluent is microcrystalline cellulose.

5. The pharmaceutical composition according to any preceding claim, wherein the at least one water insoluble diluent comprises an amount from about 40% to about 70% of the total weight of the composition.

6. The pharmaceutical composition according to any preceding claim, wherein the surfactant is selected from the group consisting of poloxamers, polyethylene glycols, polysorbates, sodium lauryl sulfate, polyethoxylated castor oil, and a hydroxylated castor oil.

7. The pharmaceutical composition according to claim 6, wherein the surfactant is Poloxamer 188.

8. The pharmaceutical composition according to any preceding claim, wherein the surfactant is in an amount from about 2.0% to about 3.5% of the total weight of the composition.

9. The pharmaceutical composition according to any preceding claim, wherein the basic agent is selected from the group consisting of alkaline hydroxides, alkaline carbonates, alkaline phosphates, basic amino acids, and Meglumine.

10. The pharmaceutical composition according to claim 9, wherein the basic agent is Meglumine.

11. The pharmaceutical composition according to any preceding claim, wherein the basic agent is in an amount from about 9% to about 12% of the total weight of the composition.

12. The pharmaceutical composition according to any preceding claim, wherein the composition further comprises from about 1.0% to about 1.5% of a binder.

13. The pharmaceutical composition according to claim 12, wherein the binder is Povidone.

14. The pharmaceutical composition according to any preceding claim, wherein the composition further comprises from about 7.5% to about 10% of a disintegrant.

15. The pharmaceutical composition according to claim 14, wherein the disintegrant is sodium starch glycolate.

16. The pharmaceutical composition according to any preceding claim, wherein the composition further comprises from about 0% to about 17% of a water soluble filler.

17. The pharmaceutical composition according to claim 16, wherein the water soluble filler is sorbitol.

18. The pharmaceutical composition according to any preceding claim, wherein the composition further comprises from about 0.5% to about 1.0% of a lubricant.

19. The pharmaceutical composition according to claim 18, wherein the lubricant is magnesium stearate.

20. The pharmaceutical composition according to any preceding claim, wherein at least 80% of telmisartan dissolves from the composition in a neutral aqueous environment within a period of 45 minutes.

21. The pharmaceutical composition according to claim 20, wherein the period is 30 minutes.

22. The pharmaceutical composition according to claim 20, wherein the period is 20 minutes.

23. The pharmaceutical composition according to any preceding claim in a pharmaceutical dosage form.

24. The pharmaceutical composition according to claim 23, wherein the dosage form is a tablet.

25. The pharmaceutical composition according to claim 23 or 24, wherein the tablet comprises an amount of a telmisartan compound selected from 20mg, 40mg, and 80mg.

26. The pharmaceutical composition according to claim 25, wherein the tablet dosage form comprises 20mg of a telmisartan compound.

27. The pharmaceutical composition according to claim 26, wherein the total weight of the tablet dosage form is from about 130mg to about 160mg.

28. A method of preparing a pharmaceutical composition comprising the following steps of mixing a disintegrant and one or more diluents in a high shear mixer to form a homogeneous mixture; preparing a granulation suspension of purified water, alcohol, a basic agent, a surfactant, and telmisartan; combining the homogeneous mixture and the granulation suspension to form a combined mixture; preparing a granulation solution of water and a binder; adding the granulation solution to the combined mixture to form a granulate; drying the formed granules; sizing the dried granules; mixing the dried granulate with a filler and a disintegrant; and adding a lubricant to form a final mixture, wherein the prepared pharmaceutical composition comprises less than 25% by weight of water soluble diluents.

29. The method according to claim 28, further comprising compressing the final mixture into tablets.

30. The method according to claim 28 or 29, wherein at least one diluent is water insoluble.

31. The method according to claim 30, wherein the water insoluble diluent is microcrystalline cellulose.

32. The method according to any of claims 28-30, wherein the total amount of water insoluble diluents is from about 40% to about 70%.

33. The method according to any of claims 28-32, wherein the disintegrant is sodium starch glycolate.

34. The method according to claim 33, wherein the amount of disintegrant used results in a pharmaceutical composition comprising about 7.5% to about 10% of a disintegrant.

35. The method according to any of claims 28-34, wherein the amount of a basic agent used results in a pharmaceutical composition comprising about 9% to about 12% of a basic agent.

36. The method according to claim 35, wherein the basic agent is Meglumine.

37. The method according to any of claims 28-36, wherein the amount of surfactant used results in a pharmaceutical composition comprising about 2.0% to about 3.5% of a surfactant.

38. The method according to claim 37, wherein the surfactant is Poloxamer 188.

39. The method according to any of claims 28-38, wherein the amount of binder used results in a pharmaceutical composition comprising about 1.0% to about 1.5% of a binder.

40. The method according to claim 39, wherein the binder is Povidone.

41. The method according to any of claims 28-40, wherein the amount of water soluble filler used results in a pharmaceutical composition comprising about 0% to about 17% of a water soluble filler.

42. The method according to claim 41, wherein the filler is sorbitol.

43. The method according to to any of claims 28-42, wherein the amount of lubricant is from about 0.5% to about 1.0%.

44. The method according to claim 43, wherein the lubricant is magnesium stearate.

45. The method according to any of claims 28-44, wherein the amount of telmisartan used results in a pharmaceutical composition comprising about 12.5% to about 15.5% of telmisartan.

46. A method of treating a patient suffering from hypertension comprising administering an effective amount of a telmisartan compound in a pharmaceutical composition comprising about 12.5% to about 15.5% of the telmisartan compound; about 2.0 to about 3.5% of a surfactant; about 9% to about 12% of a basic agent; and at least one diluent, wherein the composition comprises less than 25% of water soluble diluents.

47. The method according to claim 46, wherein the pharmaceutical composition comprises at least one water insoluble diluent.

48. The method according to claim 47, wherein the total amount of water insoluble diluents is from about 40% to about 70%.

49. The method according to claim 47, wherein the insoluble diluent is microcrystalline cellulose.
